# EUROPÄISCHE PATENTSCHRIFT

(11) **EP 1 511 437 B1**
(45) Veröffentlichungstag und Bekanntmachung des Hinweises auf die Patenterteilung: **10.12.2008**
(21) Anmeldenummer: 03735646.6
(22) Anmeldetag: 30.05.2003
(51) Int. Cl.: A61B 18/14

(54) **HOCHFREQUENZAPPLIKATIONSVORRICHTUNG**
HIGH FREQUENCY APPLICATION DEVICE
DISPOSITIF D'APPLICATION HAUTE FREQUENCE

(30) Priorität: 29.05.2002 DE 10224451
(43) Veröffentlichungstag der Anmeldung: 09.03.2005
(73) Patentinhaber: Celon AG Medical Instruments, 14513 Teltow (DE)
(72) Erfinder: DESINGER, Kai, 12157 Berlin (DE); ROGGAN, André, 12163 Berlin (DE); STEIN, Thomas, 10787 Berlin (DE)
(74) Vertreter: Eisenführ, Speiser & Partner
(86) Internationale Anmeldenummer: PCT/EP2003/006460
(87) Internationale Veröffentlichungsnummer: WO 2004/039272

(56) Entgegenhaltungen:
- EP-A- 1 002 501
- EP-A- 1 008 327
- EP-A- 1 050 279
- EP-A- 1 145 686
- WO-A-00/33753

## Beschreibung

Die Erfindung betrifft eine Hochfrequenzapplikationsvorrichtung mit einem Hochfrequenzgenerator, einer mit dem Hochfrequenzgenerator verbundenen, mindestens zwei Elektroden umfassenden Sondenanordnung und mindestens zwei Leitungen, welche die Elektroden mit den Hochfrequenzgenerator verbinden, wobei die Leitungen in einem gemeinsamen Kabel zusammengefasst sind und wenigstens über eine Teillänge des Kabels in einem definierten Abstand parallel zueinander verlaufen. Eine derartige Vorrichtung ist aus WO-A-00/33753 bekannt.

Eine in der Medizin bekannte Methode zum Behandeln von pathologisch verändertem Körpergewebe ist das elektrochirurgische, insbesondere das elektrothermische Veröden des betroffenen Gewebes. Von besonderem Interesse ist diese Methode für die Therapie von Organtumoren, z. B. Lebertumoren. Zur Verödung werden eine oder mehrere Elektroden im zu verödenden Gewebe, d. h. dem Tumorgewebe, oder in dessen unmittelbarer Nähe platziert und ein Wechselstrom zwischen den Elektroden oder einer Elektrode und einer außen am Körper fixierten, sogenannten Neutral-elektrode fließen gelassen. Fließt der Strom zwischen der Elektrode und der Neutralelektrode (ggf. auch zwischen mehreren Elektroden und einer oder mehreren Neutralelektroden), so spricht man von einer monopolaren Elektrodenanordnung. Fließt der Strom dagegen zwischen den im Gewebe befindlichen Elektroden selbst (in diesem Fall müssen mindestens zwei Elektroden in das Gewebe eingebracht sein), so spricht man von einer bipolaren Anordnung. Die zum Platzieren im Gewebe vorgesehene Elektrode ist in der Regel auf einer zur Punktion des Gewebes geeigneten Nadel, der sog. Elektrodennadel, angeordnet. In der bipolaren Anordnung kann eine Nadel mit mindestens zwei koaxial hintereinander angeordneten Elektroden Verwendung finden. Alternativ können aber auch mehrere Nadeln mit jeweils mindestens einer Elektrode verwendet werden.

Zum Veröden des Körpergewebes, insbesondere pathologisch veränderten Gewebes, wird mittels Hochfrequenzgenerator ein Stromfluss zwischen den mit dem Körpergewebe in elektrisch leitendem Kontakt stehenden, sog. aktiven Elektroden und beispielsweise einer Neutralelektrode induziert. Der elektrische Widerstand des Körpergewebes führt dabei dazu, dass der Wechselstrom in Wärme umgewandelt wird. Bei Temperaturen zwischen 50°C und 100° C kommt es zu einer massiven Denaturierung der körpereigenen Proteine und in der Folge zum Absterben der betroffenen Gewebeareale. Aufgrund der hohen Stromdichte im Bereich der aktiven Elektroden erfolgt die Erwärmung des Gewebes vorwiegend dort, wo die aktiven Elektroden mit dem Körpergewebe im elektrisch leitfähigen Kontakt stehen.

Im Interesse einer wirkungsvollen Behandlung ist es vorteilhaft, den Fortschritt der Behandlung möglichst zeitnah zu überprüfen. Zu diesem Zweck ist es wünschenswert, den Fortschritt der Tumorbehandlungen während der Behandlung mittels Kernspintomographie zu überwachen. Die Kernspintomographie eignet sich insbesondere zur Abbildung von Tumoren im Körpergewebe sowie zur Abbildung des Koagulationszustandes des Gewebes. Um Störungen der Kernspintomographen durch elektromagnetische Störfelder zu vermeiden, werden sie in besonders abgeschirmten Räumen betrieben. Elektromagnetische Störfelder würden nämlich die Bildgebung erheblich stören und so zu Artefakten in der Abbildung führen. In Extremfällen würde die Bildgebung dadurch unmöglich.

Die Hochfrequenzgeneratoren zum Erzeugen des in der elektrochirurgischen Behandlung eingesetzten Wechselstroms müssen daher aufgrund der von ihnen ausgehenden elektrischen und magnetischen Störfelder außerhalb der abgeschirmten Räume betrieben werden, was zu großen Abständen und langen Leitungen zwischen dem Hochfrequenzgenerator und den Elektroden der Körpersonden, bspw. der Elektrodennadeln, führen kann.

Aufgabe der Erfindung ist es daher, eine Hochfrequenzapplikationsvorrichtung zur Verfügung zu stellen, die für den Betrieb zusammen mit einem Kernspintomographen besonders geeignet ist, insbesondere auch dann, wenn der Kernspintomograph einen großen Abstand zwischen dem Hochfrequenzgenerator und der Sondenanordnung notwendig macht.

Dies Aufgabe wird durch eine Hochfrequenzapplikationsvorrichtung gemäß Anspruch 1 gelöst. Als Sonden sind hierbei nicht nur zum Einführen in den Körper geeignete Sonden, wie beispielsweise Katheter oder Elektrodennadeln, zu verstehen, sondern auch solche, die äußerlich am Körper anzubringen sind, wie bspw. Neutralelektroden.

Die Erfindung beruht auf den folgenden Überlegungen:

Je länger das Kabel zwischen dem Hochfrequenzgenerator und der Sondenanordnung ist, desto größer ist der Leistungsverlust im Kabel. Um den Leistungsverlust in langen Kabeln zu kompensieren, sind die Hochfrequenzgeneratoren von Hochfrequenzapplikationsvorrichtungen nach Stand der Technik derart ausgestaltet, dass sie eine höhere Leistung abgeben, als dies eigentlich zur Behandlung nötig wäre.

Die Erfindung verfolgt nun nicht den Weg, den Leistungsverlust durch Erhöhen der Leistung des Generators auszugleichen, sondern zielt darauf ab, den Leistungsverlust der Leitungen selbst zu vermindern. Gleichzeitig können auch von den Leitungen ausgehende Störungen der Bildgebung im Kernspintomographen vermindert werden. Aufgrund des verminderten Leistungsverlustes kann die Leistung, die der Hochfrequenzgenerator zusätzlich zur für die Behandlung benötigten Leistung für das Kompensieren des Leistungsverlustes in den Leitungen abgeben muss, verringert werden.

Dies wird erfindungsgemäß durch den Aufbau des Kabels, insbesondere durch die Anordnung der Leitungen zueinander, erreicht. Da also die Verbindung zwischen den aktiven Elektroden und dem Hochfrequenzgenerator sowie die Verbindung zwischen den ableitenden (passiven) Elektroden bzw. Neutralelektroden und dem Hochfrequenzgenerator in einem Kabel zusammengefasst sind, bedeutet dies, dass das Kabel mindestens zwei Leitungen besitzt.

Der Vorteil dieser Anordnung besteht darin, dass sich bspw. parasitäre Induktivitäten, die im Stand der Technik aus der häufig unachtsamen und daher gewundenen Verlegung der langen Leitungen zwischen dem Generator und dem Patienten resultieren können, durch die gegenläufigen Stromwege auf der Hin- und Rückleitung des Kabels der erfindungsgemäßen Hochfrequenzapplikationsvorrichtung kompensieren.

Das Zusammenfassen der Leitungen verringert den induktiven Widerstand der Leitungen sowie die beim Fließen des Wechselstroms vom Kabel abgestrahlte Leistung und somit den Leistungsverlust und die Leistungsdämpfung der Leitungen gegenüber nicht zusammengefassten Leitungen.

In der erfindungsgemäßen Hochfrequenzapplikationsvorrichtung kann der Hochfrequenzgenerator beim Betrieb weiter von der Sondenanordnung entfernt aufgestellt sein als bei einer Hochfrequenzapplikationsvorrichtung nach Stand der Technik, ohne dass dazu seine Leistungsabgabe gegenüber dem Generator in der Hochfrequenzapplikationsvorrichtung nach Stand der Technik erhöht werden müsste.

Andererseits kann, da der Leistungsverlust im Kabel geringer ist als bei einer Hochfrequenzapplikationsvorrichtung nach Stand der Technik mit gleich langem Kabel, die Leistungsabgabe des Generators gegenüber dem Generator in der Hochfrequenzapplikationsvorrichtung nach Stand der Technik verringert werden.

Die Vorteile der erfindungsgemäßen Hochfrequenzapplikationsvorrichtung führen insbesondere dazu, dass der Hochfrequenzgenerator selbst bei Behandlungen, die zusammen mit Kernspintomographen durchgeführt werden, die in einem großen abgeschirmten Raum untergebracht sind, außerhalb des abgeschirmten Raumes aufgestellt sein kann.

Die genannten Vorteile lassen sich in besonders hohem Maße erreichen, wenn die Leitungen im Kabel wenigstens über eine vorzugsweise mehr als die Hälfte der Gesamtlänge des Kabels messende Teillänge des Kabels in einem definierten Abstand parallel zueinander verlaufen. Dieser definierte Abstand entspricht vorzugsweise dem 2 bis 20fachen Durchmesser der Leitungen. Geeignete Abstände liegen zwischen 1 mm und 25 mm. Die Teillänge des Kabels, über die dessen Leitungen parallel zueinander verlaufen, misst vorzugsweise wenigstens 4 Meter.

Neben dem ohmschen und dem induktiven Widerstand ist auch der Wert der Kabelkapazität von Bedeutung. Die Kabelkapazität ist umso größer und damit der kapazitive Widerstand des Kabels umso kleiner, je dichter nebeneinander die beiden Leitungen im Kabel verlaufen. Ist die Kapazität zu groß, versagt bei einigen Hochfrequenzgeneratoren die zum Feststellen eines Ablösens der Neutralelektroden oder zum Feststellen des Therapieendes (z.B. wenn die Gewebeimpedanz einen bestimmten vorgegebenen Anstieg erreicht hat) nötige Impedanzmessung; sie liefert wegen der hohen Kabelkapazität und dem damit verbundenen geringen kapazitiven Widerstand einen zu niedrigen Impedanzwert, da der Wechselstrom der Impedanzmessung einen mit einem relativ niedrigen kapazitiven Widerstand verbundenen Strompfad von der einen zur anderen Leitung im Kabel vorfindet. Um eine ungestörte Impedanzmessung zu ermöglichen, ist es daher vorteilhaft, die Leitungen im Kabel in einem definierten Abstand zueinander zu führen. Durch eine geeignete Wahl des definierten Abstands lässt sich die Kabelkapazität auf einen Wert einstellen, mit dem sich die genannten Probleme vermeiden lassen. Welcher Abstand zwischen den Leitungen sich eignet, hängt vom Querschnitt der Leitungen ab. Je größer der Leitungsquerschnitt ist, desto größer sollte der Abstand zwischen den Leitungen sein. Als geeigneter Abstand erweist sich für die Leitungen, die üblicherweise für Hochfrequenzapplikationsvorrichtungen Verwendung finden, ein Abstand zwischen 1 und 25 mm, vorzugsweise zwischen 1 und 10 mm.

Vorteilhafterweise verlaufen die Leitungen am generatorseitigen Ende des Kabels getrennt voneinander, um den Anschluss an solche Hochfrequenzgeneratoren zu ermöglichen, wie sie üblicherweise in Hochfrequenzapplikationsvorrichtungen nach Stand der Technik Verwendung finden. Diese üblichen Hochfrequenzgeneratoren weisen typischerweise zwei getrennte, unterschiedliche Anschlüsse für die Leitungen auf.

Im Falle der monopolaren Anwendung kann auch am sondenseitigen Ende des Kabels eine Aufspaltung der Leitungen erfolgen, um einen getrennten Anschluss der Elektrodennadel und der Neutralelektrode zu ermöglichen.

Um den ohmschen Widerstand zu verringern, kann eine Leitung jeweils mehrere Adern umfassen. Je mehr Adern eine Leitung umfasst, desto größer ist der für den Stromfluss wirksame Querschnitt der Leitung, so dass ihr ohmscher Widerstand sinkt. Die Ausgestaltung mit mehreren Adern pro Leitung verringert jedoch nicht nur den ohmschen Widerstand der Leitung, sondern ermöglicht es auch, Adern für das Durchführen von Messungen, beispielsweise die Messung der Impedanz des Strompfades zwischen zwei Elektroden oder Temperaturmessungen, zur Verfügung zu stellen.

In einer Ausgestaltung der erfindungsgemäßen Hochfrequenzapplikationsvorrichtung sind die Leitungen und/oder die Adern miteinander verseilt, d.h. miteinander verdrillt. Das Verseilen verringert die vom Kabel abgestrahlte Leistung weiter und somit auch die Leistungsdämpfung. Zusätzlich verringert das Verseilen die Störempfindlichkeit der Leitung.

In einer alternativen Ausgestaltung der Hochfrequenzapplikationsvorrichtung verlaufen die Leitungen koaxial zueinander.

In einer Ausführungsform umfasst die Sondenanordnung eine Elektrodennadel. Zum Verwenden der Sonde im bipolaren Modus weist die Elektrodennadel mindestens zwei aktive Elektroden auf, von denen jede mit einer Leitung oder einer Ader des Kabels verbunden ist. In einer alternativen, zum Verwenden der Sonde im monopolaren Modus vorgesehenen Ausgestaltung umfasst die Sondenanordnung eine Elektrodennadel und eine äußerlich am Körper anzubringende Elektrode. Die Elektrodennadel weist in diesem Fall mindestens eine aktive Elektrode auf, wobei die aktive Elektrode und die Neutralelektrode jeweils mit einer Leitung oder Ader des Kabels verbunden sind.

Neben der Verbesserung der Leitungseigenschaften (ohmscher, kapazitiver und induktiver Widerstand) gilt es bei der Kernspintomographie, die Weiterleitung von Störsignalen durch die Leitungen zu unterdrücken. Dies ist wichtig, weil sich der Hochfrequenzgenerator außerhalb des Messraums befindet und somit in einer nicht entstörten Umgebung betrieben wird. Durch das Verbindungskabel können diese Störungen prinzipiell aufgefangen (Antenne) und in den Messraum weitergeleitet werden. Solche vom Kabel aufgenommenen elektromagnetischen Störfelder können die Bildgebung im Kernspintomographen erheblich stören.

In einer weiteren Ausgestaltung der Hochfrequenzapplikationsvorrichtung kann daher das Kabel außerhalb des Raumes, in dem sich der Kernspintomograph befindet, von einem ferromagnetischer Ring, insbesondere ein Ferrit-Kern, als Hochfrequenzdrossel umgeben sein. Als Ring sollen hierbei nicht nur geometrisch ringförmige Objekte zu verstehen sein, sondern auch all solche, die eine ovale, eckige oder unregelmäßige Form haben und eine Kabeldurchführung aufwiesen.

Mit der Hochfrequenzdrossel lassen sich Störungen herausfiltern, ohne die Kapazität des Kabels zu erhöhen.

In einer weiteren Ausgestaltung der Erfindung werden elektromagnetische Störfelder durch einen das Kabel umgebenden und elektrisch leitenden Schirm oder Mantel abgeschirmt. Der Schirm bzw. der Mantel erstreckt sich mindestens vom Generator bis an die Stelle des Kabels, an der es in den abgeschirmten Raum des Kernspintomographen eingeführt wird. Vorteilhafterweise ist der Schirm bzw. der Mantel derart ausgestaltet, dass er sich elektrisch mit der Abschirmeinrichtung des Kernspintomographen, die von einem farradayischen Käfig gebildet wird, verbinden lässt.

Eine weitere Möglichkeit zum Unterdrücken von Störsignalen, die unabhängig vom Zusammenfassen der Leitungen in einem Kabel und dem ferromagnetischen Ring vorteilhaft ist, besteht darin, eine Schalteinrichtung vorzusehen, mit der sich ein generatorseitiger Abschnitt des Kabels bzw. der Leitungen von einem sondenseitigen Abschnitt des Kabels bzw. der Leitungen trennen und wieder verbinden lässt. Durch Betätigen der Schalteinrichtung wird der generatorseitige Abschnitt vom sondenseitigen Abschnitt getrennt, so dass keine Störungen über das Kabel in den Raum des Kerspintomographen transportiert werden. Dies ist insbesondere vorteilhaft, wenn mit dem Kernspintomographen hochempfindliche Messungen vorgenommen werden sollen, die bereits durch kleinste Störsignale erheblich gestört würden.

Die Schalteinrichtung kann ein oder mehrere Relais, insbesondere Reedrelais umfassen. Alternativ ist auch eine Ausgestaltung der Schalteinrichtung mit mindestens einem mechanischen Schalter, beispielsweise einem pneumatischer Schalter, möglich.

Die Schalteinrichtung ist vorteilhafterweise derart ausgestaltet, dass sie, im Normalzustand geschlossen ist, d. h. eine leitende Verbindung zwischen dem generatorseitigen und dem sondenseitigen Abschnitt des Kabels besteht.

Zum Auslösen der Schalteinrichtung kann diese eine Signalleitung umfassen, an deren Ende ein Betätigungsschalter angeordnet ist, mit dem die Schalteinrichtung von dem Raum aus, in dem sich der Hochfrequenzgenerator befindet, zu schalten ist.

Alternativ besteht auch die Möglichkeit, die Schalteinrichtung mit einer Schnittstelle auszustatten, an die eine Steuerleitung des Kernspintomographen anschließbar ist, so dass der Kernspintomograph selbsttätig den generatorseitigen Abschnitt des Kabels vom sondenseitigen Abschnitt trennen kann, beispielsweise wenn eine hochempfindliche Messung durchzuführen ist.

In einer weiteren vorteilhaften Ausgestaltung sind das Kabel und die Sondenanordnung resterilisierbar ausgestaltet, so dass die Hochfrequenzapplikationsvorrichtung zur Behandlung eines Patienten in einer sterilen Umgebung geeignet ist.

Weitere Merkmale und Vorteile der Erfindung sind nachfolgend anhand von Ausführungsbeispielen unter Bezugnahme auf die beiliegenden Zeichnungen beschrieben. Die Ausführungsbeispiele ohne Luftpolster sind nicht Gegenstand der Erfindung.
- Figur 1: zeigt ein erstes Ausführungsbeispiel für die erfindungsgemäße Hochfrequenzapplikationsvorrichtung.
- Figur 2: zeigt eine erste Ausführungsform des Kabels zwischen dem Hochfrequenzgenerator und der Sondenanordnung.
- Figur 3: zeigt eine zweite Ausführungsform des Kabels zwischen dem Hochfrequenzgenerator und der Sondenanordnung.
- Figur 4: zeigt eine dritte Ausführungsform des Kabels zwischen dem Hochfrequenzgenerator und der Sondenanordnung.

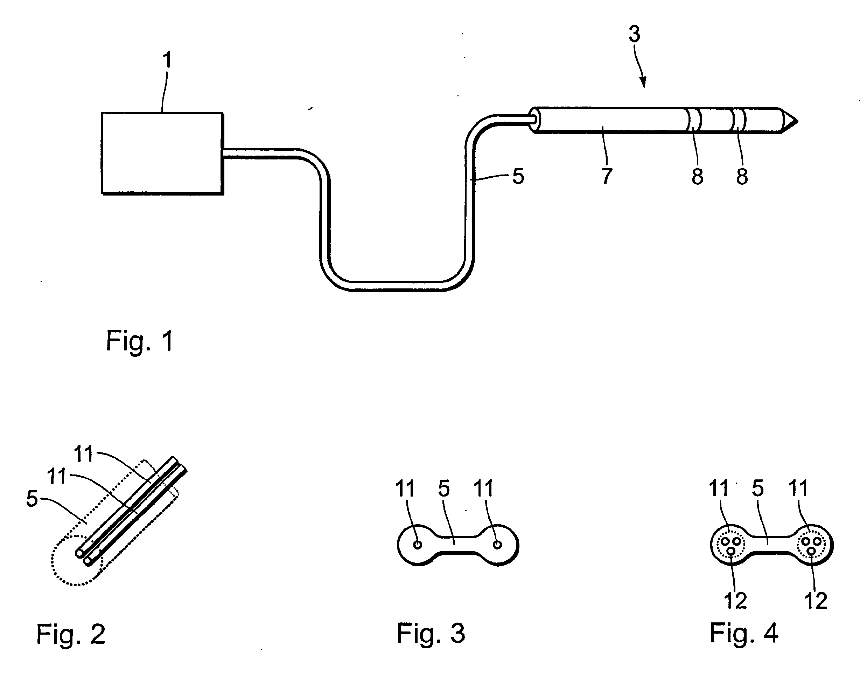

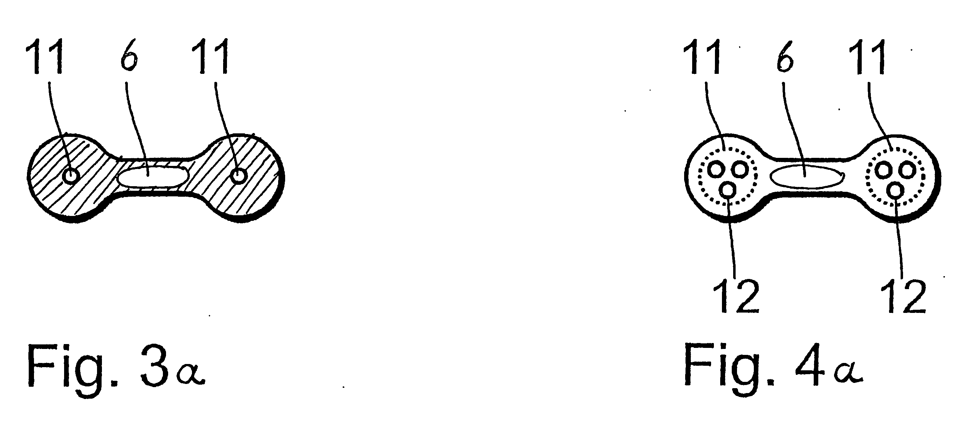

- Figuren 3a und 4a: zeigen Alternativen zu den Ausführungsformen der Kabel gemäß der Figuren 3 und 4 mit einem zusätzliche Luftpolster zwischen den Leitungen.

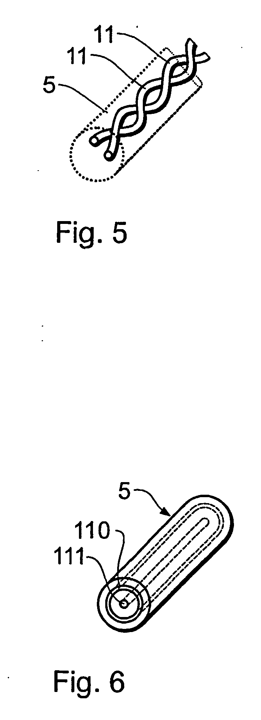

- Figur 5: zeigt eine vierte Ausführungsform des Kabels zwischen dem Hochfrequenzgenerator und der Sondenanordnung.
- Figur 6: zeigt eine fünfte Ausführungsform des Kabels zwischen dem Hochfrequenzgenerator und der Sondenanordnung.

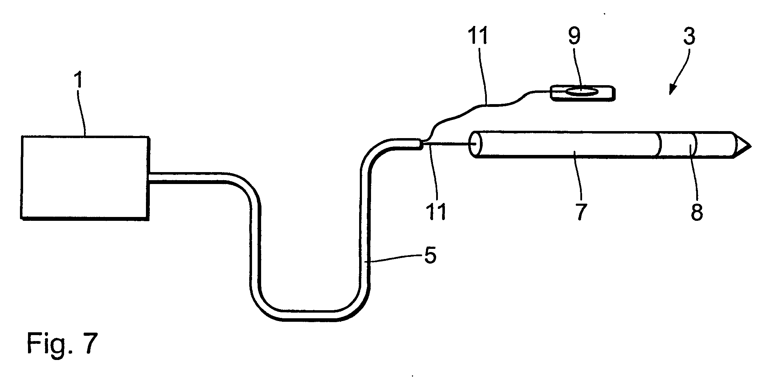

- Figur 7: zeigt ein zweites Ausführungsbeispiel für die erfindungsgemäße Hochfrequenzapplikationsvorrichtung.

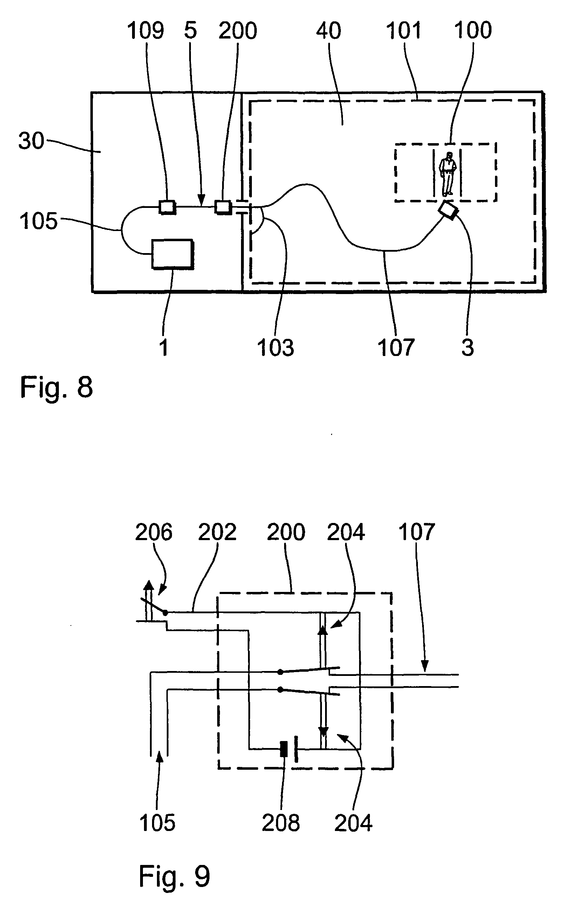

- Figur 8: zeigt das Setup der erfindungsgemäßen Hochfrequenzapplikationsvorrichtung in der Anwendung.
- Figur 9: zeigt eine am Kabel der Hochfrequenzapplikationsvorrichtung angeordnete Schalteinrichtung.

Figur 1 zeigt ein erstes Ausführungsbeispiel für die erfindungsgemäße Hochfrequenzapplikationsvorrichtung. Die Hochfrequenzapplikationsvorrichtung umfasst einen Hochfrequenzgenerator 1, eine Sondenanordnung 3 sowie ein Kabel 5, das den Hochfrequenzgenerator 1 mit der Sondenanordnung 3 verbindet.

Die Sondenanordnung 3 des ersten Ausführungsbeispiels umfasst eine zur bipolaren Anwendung ausgestaltete Elektrodennadel 7 mit einem zum Einführen in das Körpergewebe vorgesehen Abschnitt, an dessen distalem Ende zwei aktive Elektroden 8 vorhanden sind. Selbstverständlich können auch mehr als zwei aktive Elektroden 8 vorhanden sein. Die Elektrodennadel 7 weist neben dem zum Einführen in den Körper vorgesehenen Abschnitt auch einen Griffabschnitt zum Handhaben der Nadel auf (in den Figuren nicht explizit dargestellt).

Verschiedene Ausführungsformen des Kabels 5 der Hochfrequenzapplikationsvorrichtung sind in den Figuren 2 bis 6 im Detail dargestellt.

Im Inneren der in den Figuren 2 und 3 dargestellten Kabel 5 verlaufen zwei achsparallele Leitungen 11, die in definiertem Abstand zueinander angeordnet sind. Der Abstand ist so klein, dass die gewünschte Verminderung der Leistungsverluste sowie der Störungen erzielt wird, jedoch ist er mindestens so groß, dass die kapazitive Kopplung der beiden Leitungen den Betrieb der Hochfrequenzapplikationsvorrichtung nicht stört. Geeignete Abstände der beiden Leitungen 11 voneinander liegen im Bereich von 1 bis 25 mm, wobei sich Abstände zwischen 1 und 10 mm als besonders geeignet erweisen.

In der in Figur 4 dargestellten Ausführungsform des Kabels 5 umfassen die Leitungen 11 mehrere Adern 12, um bspw. den ohmschen Widerstand der Leitungen 11 zu verringern oder Messadem zur Verfügung zu stellen.

Die erfindungsgemässen Kabel der Figuren 3a und 4a entsprechen bis auf ein jeweils zwischen den Leitungen 11 angeordnetes Luftpolster 6 den Kabeln aus den Figuren 3 und 4. Durch das Luftpolster 6 lässt sich die Dielektrizitätskonstante εᵣ zwischen den Leitungen 12 dahingehend optimieren, dass die Kapazität des Kabels pro Längeneinheit sinkt.

Eine weitere Variante des Kabels 5 ist in Figur 5 dargestellt. Dieses Kabel 5 umfasst ebenfalls zwei Leitungen 11, die jedoch miteinander verseilt (verdrillt) sind. Falls eine Leitung 11 mehrere Adern 12 umfast, können die Adern 12 einer Leitung 11 ebenfalls miteinander verseilt sein.

In Figur 6 ist eine Variante des Kabels 5 dargestellt, in der die Leitungen 110 und 111 in definiertem Abstand koaxial zueinander verlaufen, wobei die eine Leitung 110 die andere Leitung 111 umgibt. Für den Abstand zwischen den beiden Leitungen 11 gelten die gleichen Überlegungen wie für das Kabel mit achsparallelen Leitungen.

Das Kabel 5 kann darüber hinaus eine Abschirmung zum Abschirmen von Störstrahlung umfassen, die insbesondere mit der Abschirmung (101 in Fig. 8) des Kernspintomographen 100 gegen elektromagnetische Störstrahlung verbindbar ist (bspw. mittels einer Verbindungsleitung 103), um beide Abschirmungen auf dasselbe Potenzial zu legen. Es ist auch möglich, jede Leitung des Kabels mit einer eigenen Abschirmung zu versehen.

Die in den Figuren 2 bis 6 dargestellten Kabel 5 können als Material, in das die Leitungen 11, 110, 111 und/oder die Adern 12 eingebettet sind, jedes in der Kabelherstellung übliche Material umfassen. Wegen ihrer Sterilisierbarkeit oder ihrer z. T. relativ niedrigen Dielektrizitätszahl sind insbesondere Polyethylen (PE), Teflon - z.B. Polytetrafluorethylen (PTFE) oder Polyfluorethylen-Propylen (FEP) -, Silikon, Neopren (Styrol Butadien Kautschuk, SBR) und Polyvinylchlorid (PVC) geeignet. Solche Materialien erlauben es, das gesamte Kabel 5 resterilisierbar herzustellen und ermöglichen damit den Betrieb der Hochfrequenzapplikationsvorrichtung in einer sterilen Umgebung.

Die Leitungen 11 oder Adern 12 des Kabels 5 sind im ersten Ausführungsbeispiel mit jeweils einer aktiven Elektrode 8 der Nadelelektrode 7 verbunden. Im Betrieb wird den aktiven Elektroden 8 von den Leitungen 11 eine Hochfrequenzspannung zugeführt, die dazu führt, dass zwischen den Elektroden 8 ein Hochfrequenzstrom durch das Körpergewebe fließt, der zur Verödung des Körpergewebes führt.

Ein zweites Ausführungsbeispiel für die erfindungsgemäße Hochfrequenzapplikationsvorrichtung ist in Figur 7 dargestellt. Diese Ausführungsform unterscheidet sich von der in Figur 1 dargestellten Ausführungsform lediglich dadurch, dass die Sondenanordnung 3 statt zur bipolaren Anwendung zur monopolaren Anwendung ausgestaltet ist. Dazu umfasst die Sondenanordnung 3 eine Nadelelektrode 7 mit einer aktiven Elektrode 8 zum Einführen in das Körpergewebe sowie eine Neutralelektrode 9 zum Anbringen an der Körperoberfläche. Sowohl die aktive Elektrode 8 als auch die Neutralelektrode 9 sind jeweils mit einer Leitung 11 oder einer Ader 12 des Kabels 5 verbunden. Die Leitungen 11 und Adern 12 werden dabei möglichst lange in definiertem Abstand zusammengefasst.

Zwar trennen sich im in Figur 7 dargestellten Ausführungsbeispiel die Leitungen 11 des Kabels 5 bereits vor der Elektrodennadel, jedoch ist es auch möglich, beide Leitungen in die Elektrodennadel, insbesondere in deren Griffabschnitt, einzuführen und sie erst dort zu trennen. In diesem Fall weist die Elektrodennadel, insbesondere der Griffabschnitt der Elektrodennadel, einen Anschluss auf, über den die Neutralelektrode mittels einer Leitung mit der Elektrodennadel 7 verbunden werden kann, so dass sie von der Elektrodennadel 7 mit Strom zu versorgen ist.

In der Anwendung der Hochfrequenzapplikationsvorrichtung wird den Elektroden 8, 9 von den Leitungen 11 eine Hochfrequenzspannung zugeführt, so dass zwischen der aktiven Elektrode 8 und der Neutralelektrode 9 ein Hochfrequenzstrom durch das Körpergewebe fließt, der zur Verödung des Tumorgewebes führt.

Ein Set-up für die Anwendung der erfindungsgemäßen Hochfrequenzapplikationsvorrichtung ist in Figur 8 dargestellt. Figur 8 zeigt einen Kernspintomographen 100, der sich in einem mit einem Farradayischen Käfig 101 als Abschirmeinrichtung gegen elektromagnetische Störstrahlung versehenen Behandlungsraum 40 befindet. Im Behandlungsraum 40 befinden sich außerdem der größte Teil des sondenseitigen Abschnitts 107 des Kabels 5, sowie die Sondenanordnung 3 der Hochfrequenzapplikationsvorrichtung. Die übrigen Komponenten der Hochfrequenzapplikationsvorrichtung, insbesondere der Hochfrequenzgenerator 1 und der generatorseitige Abschnitt 105 des Kabels 5 befinden sich hingegen in einem Betriebsraum 30, der dem Behandlungsraum 40 benachbart ist.

Ist das Kabel 5 mit einer Abschirmung gegen elektromagnetische Störstrahlung versehen, so weist die Abschirmung ein Verbindungskabel 103 auf, mit dem sie mit dem Farradayischen Käfig 101 des Behandlungsraums 40 verbunden ist, um sowohl den Farradayischen Käfig 101 als auch die Abschirmung auf ein gemeinsames Potential zu legen. Die Abschirmung des Kabels 5 erstreckt sich mindestens vom Generator 1 bis des Kabels 5 erstreckt sich mindestens vom Generator 1 bis zur Durchführung, durch die das Kabel 5 in den Behandlungsraum 40 eintritt. Typischerweise wird sich die Abschirmung jedoch über das gesamte Kabel 5 erstrecken, da dies einfacher herzustellen ist.

Am Kabel 5 kann außerdem mindestens eine Hochfrequenzdrossel, z.B. ein Ferrit-Kern 109, zum unterdrücken von Störsignalen auf den Leitungen 11 angeordnet sein.

Weiterhin kann zwischen dem generatorseitigen Abschnitt 105 und dem sondenseitigen Abschnitt 107 des Kabels 5 eine Schalteinrichtung 200 angeordnet sein, mit der sich beide Abschnitte elektrisch voneinander trennen lassen. Sie kann entweder im Behandlungsraum 40 oder im Betriebsraum 30 angeordnet sein, sollte sich jedoch vorzugsweise in der Nähe der beide Räume miteinander verbindenden Durchführöffnung zum Durchführen des Kabels befinden.

Die Schalteinrichtung 200 ist in Figur 9 im Detail dargestellt. Es sind der generatorseitige Abschnitt 105, sowie der sondenseitige Abschnitt 107 des Kabels 5 zu erkennen. Zwischen den jeweiligen Leitungen der beiden Abschnitte befinden sich Schalter 204, mit denen die Leitungen der beiden Abschnitte voneinander getrennt oder miteinander verbunden werden können.

Die Betätigung der Schalter 204 erfolgt über einen Betätigungsschalter 206, der über eine Signalleitung 202 mit einem eine Batterie 208 unfassenden Stromkreis verbunden ist und dessen Betätigung das Öffnen der Schalter 204 auslöst.

Optional können alle in Figur 9 dargestellten Leitungen bzw. Leitungsabschnitte mit ferromagnetischen Ringen, insbesondere Ferrit-Kernen, zum Unterdrücken von Störsignalen ausgestattet sein.

Als Schalter 204 können Relais, insbesondere Reedrelais zur Anwendung kommen. Alternativ ist es jedoch auch möglich mechanische Schalter, beispielsweise pneumatische Schalter, zu verwenden.

Die Signalleitung 202 kann, wie in Figur 9 dargestellt, zu einem Betätigungsschalter 206 führen, mit dessen Hilfe das Bedienpersonal im Betriebsraum 30 das Zuführen des Hochfrequenzstroms an die Sondenanordnung 3 unterbrechen kann, wenn mit dem Kernspintomographen 100 Messungen vorgenommen werden sollen, die sehr empfindlich auf Störstrahlung reagieren.

Alternativ kann das Verbindungskabel 202 auch zum Anschließen an eine Schnittstelle des Kernspintomographen 100 geeignet sein, so dass dieser selbständig die Zufuhr von Hochfrequenzstrom an die Sondeneinrichtung 3 unterbrechen kann, wenn eine besonders empfindliche Messung ansteht.

## Patentansprüche

1. Hochfrequenzapplikationsvorrichtung mit einem Hochfrequenzgenerator (1), einer mit dem Hochfrequenzgenerator (1) verbundenen, mindestens zwei Elektroden (8, 9) umfassenden Sondenanordnung (3) und mindestens zwei Leitungen (11; 110, 111), welche die Elektroden (8, 9) mit dem Hochfrequenzgenerator (1) verbinden,
wobei
die Leitungen (11; 110, 111) in einem gemeinsamen Kabel (5) zusammengefasst sind und wenigstens über eine Teillänge des Kabels (5) in einem definierten Abstand, der zwischen 1 mm und 25 mm beträgt, parallel zueinander verlaufen, und wobei auf der Teillänge des Kabels auf der die Leitungen (11,110,111) parallel zueinander verlaufen, ein Luftpolster (6) zwischen den Leitungen (11,110,111) innerhalb des Kabels (5) angeordnet ist.

2. Hochfrequenzapplikationsvorrichtung nach Anspruch 1, **dadurch gekennzeichnet, dass** die Leitungen (11; 110, 111) am generatorseitigen Ende des Kabels (5) getrennt verlaufen.

3. Hochfrequenzapplikationsvorrichtung nach Anspruch 1 oder 2, **dadurch gekennzeichnet, dass** eine Leitung (11) mehrere Adern (12) umfasst.

4. Hochfrequenzapplikationsvorrichtung nach einem der Ansprüche 1 bis 3, **dadurch gekennzeichnet, dass** die Leitungen (11) und/oder die Adern (12) miteinander verseilt sind.

5. Hochfrequenzapplikationsvorrichtung nach einem der Ansprüche 1 bis 3, **dadurch gekennzeichnet, dass** die Leitungen (110, 111) koaxial zueinander verlaufen.

6. Hochfrequenzapplikationsvorrichtung nach einem der Ansprüche 1 bis 5, **dadurch gekennzeichnet, dass** die Sondenanordnung (3) eine Elektrodennadel (7) umfasst.

7. Hochfrequenzapplikationsvorrichtung nach Anspruch 6, **dadurch** gekenntzeichnet, dass die Elektrodennadel (7) mindestens zwei aktive Elektroden (8) umfasst.

8. Hochfrequenzapplikationsvorrichtung nach einem der Ansprüche 1 bis 5, **dadurch gekennzeichnet, dass** die Sondenanordnung (3) eine Elektrodennadel (7) sowie eine äußerlich am Körper anzubringende Neutralelektrode (9) umfasst.

9. Hochfrequenzapplikationsvorrrichtung nach Anspruch 6, **dadurch gekennzeichnet, dass** die Elektrodennadel (7) mindestens eine aktive Elektrode (8) umfasst.

10. Hochfrequenzapplikationsvorrichtung nach einem der vorangehenden Ansprüche, **dadurch gekennzeichnet, dass** am Kabel (5) ein ferromagnetischer Ring angebracht ist.

11. Hochfrequenzapplikationsvorrichtung nach einem der vorangehenden Ansprüche, **dadurch gekennzeichnet, dass** das Kabel (5) mit einem elektrisch leitenden Schirm oder Mantel versehen ist.

12. Hochfrequenzapplikationsvorrichtung nach Anspruch 11, **dadurch gekennzeichnet, dass** der Schirm bzw. der Mantel eine Verbindung (103) umfasst, über die er elektrisch mit einer Abschirmeinrichtung (101) eines Kernspintomographen (100) zu verbinden ist.

13. Hochfrequenzapplikationsvorrichtung nach einem der vorangehenden Ansprüche
**dadurch gekennzeichnet, dass**
die Leitungen (11; 110, 111) einen generatorseitigen Abschnitt (105) und einen sondenseitigen Abschnitt (107) aufweisen, zwischen denen eine Schalteinrichtung (200) zum Trennen und Verbinden des generatorseitigen und des sondenseitigen Abschnitts angeordnet ist.

14. Hochfrequenzapplikationsvorrichtung nach Anspruch 13, **dadurch gekennzeichnet, dass** die Schalteinrichtung (200) einen elektrischen Schalter (204) umfasst.

15. Hohfrequenzapplikationsvorrichtung nach Anspruch 14, **dadurch gekennzeichnet, dass** der elektrische Schalter (204) ein Reedrelais ist.

16. Hochfrequenzapplikationsvorrichtung nach einem der Ansprüche 13 bis 15, **dadurch gekennzeichnet, dass** die Schalteinrichtung (200) einen mechanischen Schalter umfasst.

17. Hochfrequenzapplikationsvorrichtung nach einem der Ansprüche 13 bis 15, **dadurch gekennzeichnet, dass** die Schalteinrichtung (200) eine Signalleitung (202) und einen Betätigungsschalter (206) umfasst, die derart angeordnet und ausgestaltet sind, dass das Trennen und Verbinden in dem Raum erfolgen kann, in dem sich der Hochspannungsgenerator (1) befindet.

18. Hochfrequenzapplikationsvorrichtung nach einem der Ansprüche 13 bis 17, **dadurch gekennzeichnet, dass** die Schalteinrichtung (200) eine Schnittstelle zum Anschließen einer Steuerleitung (202) an einen Kenspintomographen (900) umfasst.

19. Hochfrequenzapplikationsvorrichtung nach einem der vorangehenden Ansprüche, **dadurch gekennzeichnet, dass** die Sondenanordnung (3) und das Kabel (5) resterilisierbar ausgestaltet sind.

## Claims

1. High frequency application apparatus comprising a high frequency generator (1), a probe arrangement (3) which is connected to the high frequency generator (1) and which comprises at least two electrodes (8, 9), and at least two lines (11; 110, 111), which connect the electrodes (8, 9) to the high frequency generator (1),
wherein the lines (11; 110, 111) are combined together in a common cable (5) and run in parallel at least over part of the length of the cable (5) at a defined distance apart, which is between 1 mm and 25 mm, and whereby on part of the length of the cable on which the lines (11; 110; 111) run parallel to one another an air cushion (6) is arranged between the lines (11; 110; 111) inside the cable (5).

2. High frequency application apparatus according to claim 1, **characterised in that** the lines (11; 110, 111) run separately at the generator-side end of the cable (5).

3. High frequency application apparatus according to claim 1 or 2, **characterised in that** a line (11) comprises several wires (12).

4. High frequency application apparatus according to one of claims 1 to 3, **characterised in that** the lines (11) and/or the wires (12) are twisted together.

5. High frequency application apparatus according to one of claims 1 to 3, **characterised in that** the lines (110, 111) run coaxially relative to one another.

6. High frequency application apparatus according to one of claims 1 to 5, **characterised in that** the probe arrangement (3) comprises an electrode needle (7).

7. High frequency application apparatus according to claim 6, **characterised in that** the electrode needle (7) comprises at least two active electrodes (8).

8. High frequency application apparatus according to one of claims 1 to 5, **characterised in that** the probe arrangement (3) comprises an electrode needle (7) and a neutral electrode (9) to be applied externally onto the body.

9. High frequency application apparatus according to claim 6, **characterised in that** the electrode needle (7) comprises at least one active electrode (8).

10. High frequency application apparatus according to one of the preceding claims, **characterised in that** a ferromagnetic ring is mounted on the cable (5).

11. High frequency application apparatus according to one of the preceding claims, **characterised in that** the cable (5) is provided with an electrically conductive shield or casing.

12. High frequency application apparatus according to claim 11, **characterised in that** the shield or the casing includes a connection (103), by means of which it is electrically connected to a shielding device (101) of a nuclear magnetic resonance tomograph (100).

13. High frequency application apparatus according to one of the preceding claims, **characterised in that** the lines (11; 110, 111) have a generator-side section (105) and a probe-side section (107), between which a switching device (200) is arranged for separating and connecting the generator-side and the probe-side section.

14. High frequency application apparatus according to claim 13, **characterised in that** the switching device (200) comprises an electric switch (204).

15. High frequency application apparatus according to claim 14, **characterised in that** the electric switch (204) is a reed relay.

16. High frequency application apparatus according to one of claims 13 to 15, **characterised in that** the switching device (200) includes a mechanical switch.

17. High frequency application apparatus according to one of claims 13 to 15, **characterised in that** the switching device (200) includes a signal line (202) and an actuating switch (206), which are arranged and configured such that the separation and connection can take place in the space in which the high-voltage generator (1) is located.

18. High frequency application apparatus according to one of claims 13 to 17, **characterised in that** the switching device (200) comprises an interface for connecting a control line (202) to a nuclear magnetic resonance tomograph (100).

19. High frequency application apparatus according to one of the preceding claims, **characterised in that** the probe arrangement (3) and the cable (5) are designed so as to be able to be resterilised.

## Revendications

1. Dispositif d'application haute fréquence comportant un générateur haute fréquence (1), un dispositif de sonde (3) comportant au moins deux électrodes (8, 9) et relié au générateur haute fréquence (1) et au moins deux lignes (11 ; 110, 111), qui relient les électrodes (8, 9) au générateur haute fréquence,
dans lequel
les lignes (11 ; 110, 111) sont réunies en un câble commun (5) et s'étendent parallèlement les unes aux autres au moins sur une longueur partielle du câble (5) selon une distance définie, qui se trouve entre 1 mm et 25 mm, et sur la longueur partielle du câble, sur laquelle les lignes (11 ; 110, 111) s'étendent parallèlement les unes aux autres, est disposé un coussin d'air (6) entre les lignes (11 ; 110, 111) à l'intérieur du câble (5).

2. Dispositif d'application haute fréquence selon la revendication 1, **caractérisé en ce que** les lignes (11 ; 110, 111) s'étendent de façon séparée à l'extrémité du câble côté générateur (5).

3. Dispositif d'application haute fréquence selon la revendication 1 ou 2, **caractérisé en ce qu'**une ligne (11) comporte plusieurs brins (12).

4. Dispositif d'application haute fréquence selon l'une des revendications 1 à 3, **caractérisé en ce que** les lignes (11) et/ou les brins (12) sont toronnés les uns avec les autres.

5. Dispositif d'application haute fréquence selon l'une des revendications 1 à 3, **caractérisé en ce que** les lignes (110, 111) s'étendent coaxialement les unes par rapport aux autres.

6. Dispositif d'application haute fréquence selon l'une des revendications 1 à 5, **caractérisé en ce que** l'agencement de sonde (3) comprend une électrode-aiguille (7).

7. Dispositif d'application haute fréquence selon la revendication 6, **caractérisé en ce que** l'électrode-aiguille (7) comprend au moins deux électrodes actives (8).

8. Dispositif d'application haute fréquence selon l'une des revendications 1 à 5, **caractérisé en ce que** l'agencement de sonde (3) comprend une électrode-aiguille (7) ainsi qu'une électrode neutre (9) à appliquer sur le corps à l'extérieur.

9. Dispositif d'application haute fréquence selon la revendication 6, **caractérisé en ce que** l'électrode-aiguille (7) comprend au moins une électrode active (8).

10. Dispositif d'application haute fréquence selon l'une quelconque des revendications précédentes, **caractérisé en ce qu'**un anneau ferromagnétique est disposé dans le câble (5).

11. Dispositif d'application haute fréquence selon l'une des revendications précédentes, **caractérisé en ce que** le câble (5) est équipé d'un blindage ou d'une enveloppe électriquement conductrice.

12. Dispositif d'application haute fréquence selon la revendication 11, **caractérisé en ce que** la gaine ou l'enveloppe comprend une connexion (103), par l'intermédiaire de laquelle elle peut être reliée électriquement au dispositif de blindage (101) d'un dispositif de tomographie par résonance magnétique (100).

13. Dispositif d'application haute fréquence selon l'une des revendications précédentes, **caractérisé en ce que** les lignes (11 ; 110, 111) présentent un tronçon côté générateur (105) et un tronçon côté sonde (107), entre lesquels est disposé un mécanisme de commutation (200) pour séparer et relier les tronçons côté générateur et côté sonde.

14. Dispositif d'application haute fréquence selon la revendication 13, **caractérisé en ce que** le mécanisme de commutation (200) comprend un interrupteur électrique (204).

15. Dispositif d'application haute fréquence selon la revendication 14, **caractérisé en ce que** l'interrupteur électrique (204) est un relais Reed.

16. Dispositif d'application haute fréquence selon l'une des revendications 13 à 15, **caractérisé en ce que** le mécanisme de commutation (200) comprend un interrupteur mécanique.

17. Dispositif d'application haute fréquence selon l'une des revendications 13 à 15, **caractérisé en ce que** le mécanisme de couplage (200) comprend une ligne signal (202) et un interrupteur de commande (206), qui sont disposés et agencés de sorte que la séparation et la connexion peuvent être effectués dans l'espace, où se trouve le générateur haute tension (1).

18. Dispositif d'application haute fréquence selon l'une des revendications 13 à 17, **caractérisé en ce que** le mécanisme de commutation (200) comprend une interface pour relier une ligne de commande (202) au dispositif de tomographie par résonance magnétique (100).

19. Dispositif d'application haute fréquence selon l'une des revendications précédentes, **caractérisé en ce que** l'agencement de sonde (3) et le câble (5) peuvent être restérilisés.
